(19) Europäisches Patentamt
European Patent Office
Office européen des brevets

(11) Numéro de publication: **0 158 211 B1**

(12) **FASCICULE DE BREVET EUROPEEN**

(45) Date de publication de fascicule du brevet: **26.08.92**

(51) Int. Cl.[5]: **C07D 211/90**, C07D 405/04, A61K 31/445

(21) Numéro de dépôt: **85103541.0**

(22) Date de dépôt: **25.03.85**

(54) **Nouveaux dérivés de 1,4 dihydropyridine, leur procédé de préparation, et leur application comme médicaments.**

(30) Priorité: **27.03.84 ES 531033**
**04.10.84 ES 536537**

(43) Date de publication de la demande:
**16.10.85 Bulletin 85/42**

(45) Mention de la délivrance du brevet:
**26.08.92 Bulletin 92/35**

(84) Etats contractants désignés:
**AT BE CH DE FR GB IT LI LU NL SE**

(56) Documents cités:
**DE-A- 2 117 573**
**US-A- 3 923 818**
**US-A- 3 932 646**

**NAUNYN-SCHMIEDEBERG'S ARCH. PHARMACOL. 310, 1979, pages 69-78, R. RODENKIRCHEN et al.: "Structure-Activity Studies on Nifedipine in Isolated Cardiac Muscle"**

**SYNTHESIS, no. 9, 1983, page 761; T. WATANABE et al.: "An Efficient Procedure for the Hantzsch Dihydropyridine Synthesis"**

(73) Titulaire: **LABORATORIOS DELAGRANGE S.A.**
**Avenida de la Industria, 17 Apartado 101**
**E-28100 Alcobendas (Madrid)(ES)**

(72) Inventeur: **Verde Casanova, Maria Jose**
**Arturo Baldesano no 5**
**E-28043 Madrid(ES)**
Inventeur: **Galiano Ramos, Joaquin Alvaro**
**Tebas no 23**
**E-Las Rozas (Madrid)(ES)**

(74) Mandataire: **Glawe, Delfs, Moll & Partner Patentanwälte**
**Rothenbaumchaussee 58 Postfach 2570**
**W-2000 Hamburg 13(DE)**

EP 0 158 211 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEMICAL ABSTRACTS, vol. 75, no. 22, 29th November, 1971, page 415, abstract no. 135519v Columbus, Ohio, US; A. KAMAL et al.: " Comparative studies of PMR spectra of some dihydropyridines. III."; & PAK.J.SCI.IND.RES. 1971, 14(1-2), 11-14

BULL.SOC.CHIM.BELG., vol. 84, no. 8-9, 1975, pages 855-859; R.A. DOMMISSE et al.: "U.V. and I.R. spectroscopic studies of 4-acryl-3,5-diethoxycarbonyl-2,6-dimethyl-1,4--dihydropyridines"

DEUTSCHE APOTHEKER ZEITUNG 104, vol. 3, 1983, pages 139-146; R. MANNHOLD et al..: "Pharmakologische Differenzierung von Calcium-Antagonisten"

## Description

La présente invention concerne une nouvelle série de composés qui ont une puissante activité antagoniste du calcium et sont utiles dans le traitement de l'hypertension, de l'angine de poitrine et d'autres troubles cardio-vasculaires.

Les composés de l'invention sont apparentés chimiquement aux inhibiteurs des canaux calciques du groupe des 1,4-dihydropyridines et exercent un effet relaxant sur le muscle cardiaque et les muscles lisses vasculaires.

Les inhibiteurs des canaux calciques forment une famille de composés très importante, de structures chimiques variées, utiles dans le traitement des troubles cardio-vasculaires.

Les dihydropyridines représentent une nouvelle classe d'antagonistes du calcium qui ont une activité relaxante des muscles lisses.

On connaissait déjà, en particulier d'après les brevets US-A-3932642 et DE-A-2117573, des dérivés de 2,6-diméthyl 4-phényl 1,4-dihydropyridine comportant des groupes esters en positions 3 et 5 et substitués sur le groupe phényle par des groupes alcoxy.

La présente invention concerne de nouvelles dihydropyridines comportant, en position 4, un groupe phényle substitué par un groupe 2,3-méthylènedioxy ou 2,3-éthylènedioxy, qui ont des propriétés antiangineuses, antihypertensives et vasodilatatrices.

Les composés de l'invention sont représentés par la formule (I) :

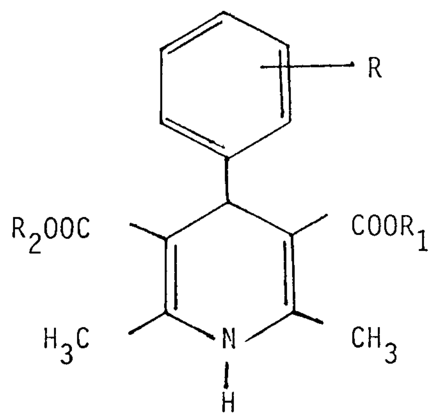

(I)

dans laquelle :

- R est un groupe 2,3-méthylènedioxy ou 2,3-éthylènedioxy, et
- $R_1$ et $R_2$ sont des groupes méthyle ou bien sont différents l'un de l'autre et choisis parmi les groupes méthyle, éthyle, isopropyle et isobutyle.

La présente invention concerne également un procédé de préparation des composés de formule (I). Ce procédé consiste à traiter un benzaldéhyde substitué de formule (II) :

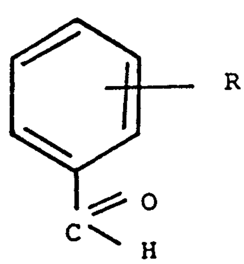

(II)

dans laquelle R est défini comme précédemment, par un ester de l'acide acétoacétique, de formule (III) :

$H_3C - CO - CH_2 - COOR_1$ (III)

dans laquelle $R_1$ est défini comme précédemment, puis à traiter l'ester de l'acide $\alpha$-acétyl -$\beta$-(phényl

substitué) acrylique , de formule (IV) ,

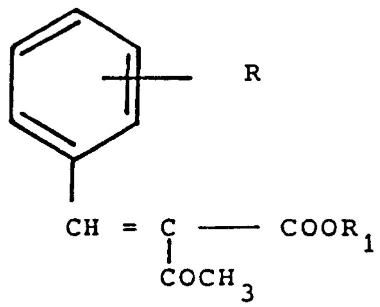

(IV)

ainsi obtenu, par un ester de l'acide 3-amino crotonique , de formule (V) :

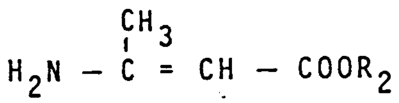

(V)

dans laquelle $R_2$ est défini comme précédemment.

La présente invention concerne aussi les compositions pharmaceutiques contenant un composé de formule (I), qui peuvent être administrées par voie orale, rectale, nasale ou sublinguale ou par injection. Ces compositions pharmaceutiques sont formées du principe actif, éventuellement sous forme de sel pharmaceutiquement acceptable, en association avec un excipient qui peut être un solide, un semi-solide ou un liquide ou une gélule comestible.

En général, le principe actif constitue 0,1% à 99% en poids de la composition, par exemple 0,5 à 10% pour les compositions injectables et 10 à 80% pour les compositions administrées par voie orale.

Pour préparer les compositions pharmaceutiques sous forme de doses unitaires à usage oral, le principe actif peut être mélangé à un excipient solide, pulvérulent, tel que lactose, saccharose, sorbitol, un amidon tel que amidon de maïs, amylopectine, agar, un dérivé de cellulose, la polyvinylpyrrolidone ou la gélatine.

On peut également ajouter des lubrifiants tels que stéarate de magnésium ou de calcium, Carbowax ou d'autres cires de polyéthylèneglycol:

On prépare ensuite les comprimés et les dragées par compression du mélange.

Dans le cas des dragées,les noyaux sont enrobés, par exemple à l'aide de solutions concentrées de sucre qui peuvent contenir de la gomme arabique, du talc et/ou du dioxyde de titane ou bien à l'aide d'agents filmogènes dissous dans un solvant organique volatil.

On peut ajouter des colorants à ces enrobages pour distinguer par exemple les différents principes actifs.

Pour préparer les gélules molles, on peut dissoudre le principe actif dans une huile telle que l'huile d'olive, l'huile de sésame ou l'huile d'arachide.

Les gélules dures peuvent contenir des granules de principe actif et des excipients solides pulvérulents tels que lactose, saccharose, amidons, dérivés de cellulose, polyvinylpyrrolidone ou gélatine. On peut également ajouter des lubrifiants tels le stéarate de magnésium ou l'acide stéarique.

Les composés de l'invention peuvent également être présentés sous des formes à libération prolongée, en choisissant les excipients appropriés.

On peut utiliser différentes méthodes de contrôle de libération : microgranules ou particulesenrobés, principe actif enrobé dans unematrice ou formes peu solubles.

Les compositions liquides à usage oral peuvent se présenter sous forme d'élixirs, sirops ou suspensions. Les solutions peuvent contenir par exemple, environ 0,1 à 10% en poids de principe actif, du sucre et un mélange d'alcool, d'eau, de glycérol et de propylèneglycol, éventuellement un arôme et de la saccharine et/ou de la carboxyméthyl-cellulose ou de la pectine comme agent de dispersion.

Pour les compositions à usage parentéral, les composés susceptibles de former des sels avec des acides tels l'acide chlorhydrique, phosphorique, tartrique ou d'autres acides minéraux ou organiques, peuvent être mis en solution aqueuse, à des concentrations de l'ordre de 0,1-0,5%. Des agents stabilisants et/ou des substances tampons peuvent éventuellement être ajoutés.

Les doses unitaires peuvent par exemple être présentées sous forme d'ampoules ou de flacons.

Les doses administrées sont variables et dépendent de différents facteurs, en particulier des besoins individuels de chaque patient.

Par voie orale, le dosage journalier est de préférence de 10 à 50 mg, en une à trois prises.

Pour l'administration parentérale, le dosage préférentiel est de 1 à 10 mg.

Les exemples suivants décrivent la préparation des composés de l'invention.

Exemple 1 : 1,4-dihydro-2,6-diméthyl-4-(2',3'-méthylènedioxyphényl) 3,5-pyridine dicarboxylate de méthyle et d'éthyle.

a) 2,3-méthylènedioxy benzaldéhyde.

On a dissous 69 g (0,5 mol.) de 2,3-dihydroxybenzaldéhyde dans 825 ml de diméthylformamide puis ajouté 144,9 g de fluorure de potassium.

On a refroidi le mélange dans un bain de glace puis ajouté lentement 96 g de dibromométhane, tout en agitant vigoureusement.

Le mélange a ensuite été chauffé à 110-120° C pendant 2 heures. Après refroidissement, la solution a été filtrée sur filtre de verre et le résidu lavé avec 300 ml de chloroforme.

Les solvants ont été éliminés sous vide donnant un produit huileux qui a été distillé sous pression réduite (5 mm Hg).

On a obtenu 54,5 g (73 %) d'une huile visqueuse (P.E. = 109° C, 5 mm Hg) qui s'est solidifiée lentement.

b) α-acétyl-β-(2,3-méthylènedioxyphényl)-acrylate d'éthyle.

Méthode 1 : Dans un ballon relié à un séparateur Dean-Stark, on a introduit 36 g de 2,3-méthylènedioxy benzaldéhyde et 31,2 g d'acétoacétate d'éthyle dans 17 ml de benzène.

On a chauffé le mélange à 60-70° C puis ajouté 0,96 ml de pipéridine et 3,84 ml d'acide acétique glacial. La solution obtenue a été chauffée au reflux pendant 2 heures jusqu'à ce qu'il n'y ait plus de séparation d'eau.

Le solvant a été éliminé sous vide, donnant un composé huileux qui s'est solidifié et a été recristallisé dans l'éthanol.

Rendement : 55 g (85%). P.F. : 97-100° C.

Méthode 2 : On a dissous 5 g de 2,3-méthylènedioxy benzaldéhyde, 4,5 g d'acétoacétate d'éthyle, 0,13 ml de pipéridine et 0,39 ml d'acide acétique glacial dans 50 ml d'éthanol absolu.

Le mélange a été chauffé au reflux pendant 2 heures puis le solvant évaporé presque jusqu'à siccité. On a rajouté 50 ml d'éthanol puis chauffé de nouveau au reflux pendant 2 heures. Le solvant a été éliminé sous vide. Le produit huileux obtenu, qui s'est solidifié après une période de repos à 4° C, a été recristallisé dans l'éthanol.

Rendement : 7,3 g (83 %). P.F. = 97 - 100° C.

c) 1,4-dihydro-2,6-diméthyl-4-(2',3'-méthylènedioxyphényl)-3,5-pyridinedicarboxylate de méthyle et d'éthyle.

On a dissous 56 g de α-acétyl-β-(2,3-méthylènedioxyphényl) acrylate d'éthyle dans 250 ml d'isopropanol chaud, puis ajouté 24 g d'aminocrotonate de méthyle.

Le mélange a été agité pendant 48 heures à 40° C puis le solvant a été éliminé sous vide.

Le solide obtenu a été lavé au diisopropyl éther puis recristallisé deux fois dans l'éthanol.

Rendement : 55 g (72%). P.F. = 166,5° C.

Exemple 2 : 1,4-dihydro-2,6-diméthyl-4-(2',3'-éthylènedioxyphényl) -3,5-pyridinedicarboxylate de méthyle et d'éthyle.

a) 2,3-éthylènedioxybenzaldéhyde.

On a dissous 5,52 g (0,04 mol.) de 2,3-dihydroxybenzaldéhyde dans 75 ml de diméthylformamide puis ajouté 11,6 g de fluorure de potassium.

On a refroidi le mélange dans un bain de glace puis ajouté lentement 7,5 g de 1,2-dibromoéthane, tout en agitant vigoureusement.

Le mélange a ensuite été chauffé pendant 2 heures à 110-120° C. Après refroidissement, la solution a été filtrée sur filtre de verre. Le résidu solide a été lavé au chloroforme puis les filtrats ont été dilués par

100 ml d'eau puis extraits au chloroforme. La phase organique a été décantée, lavée à la soude (1 N) et à l'eau, séchée sur sulfate de magnésium anhydre puis évaporée sous vide. Le produit huileux obtenu a été utilisé sans autre purification à l'étape suivante.

Rendement ; 4,5 g (70%).

b) $\alpha$-acétyl-$\beta$-(2,3-éthylènedioxyphényl) acrylate d'éthyle.

Dans un ballon relié à un séparateur Dean-Stark contenant du benzène anhydre, on a introduit 4,5 g de 2,3 - éthylènedioxybenzaldéhyde, 3,5 g d'acétoacétate d'éthyle et 10 ml de benzène.

Le mélange a été chauffé doucement jusqu'à dissolution totale, puis on a ajouté 0,12 ml de pipéridine et 0,4 ml d'acide acétique glacial. La solution a été chauffée au reflux pendant 2 heures. Après refroidissement, le mélange a été dilué par du benzène puis lavé 3 fois avec 25 ml d'acide chlorhydrique à 5 %, du bicarbonate de sodium à 5% et de l'eau. La phase organique a été décantée, séchée sur sulfate de magnésium anhydre puis le solvant a été évaporé sous vide.

Le produit huileux obtenu qui s'est solidifé lentement, a été recristallisé dans l'éthanol.

Rendement : 6,5 g (85%).

c) 1,4-dihydro-2,6-diméthyl-4-(2',3'-éthylènedioxyphényl)-3,5-pyridinedicarboxylate de méthyle et d'éthyle.

On a dissous 6,5 g de $\alpha$-acétyl-$\beta$-(2,3-éthylènedioxyphényl)acrylate d'éthyle dans 50 ml d'isopropanol chaud puis ajouté 2,7 g d'aminocrotonate de méthyle.

Le mélange a été agité pendant 48 heures à 40 °C puis le solvant a été éliminé sous vide, donnant un produit huileux qui a été recristallisé dans l'éthanol.

Rendement : 6,8 g (77 %). P.F. = 169 °C.

Exemple 3 : 1,4-dihydro-2,6-diméthyl-4-(2',3'-méthylènedioxyphényl) - 3,5-pyridine dicarboxylate de méthyle.

a) $\alpha$-acétyl -$\beta$-(2,3-méthylènedioxyphényl) - acrylate de méthyle.

Dans un ballon relié à un séparateur Dean-Stark, on a introduit 28 g de 2,3-méthylènedioxy-benzaldéhyde et 21,6 g d'acétoacétate de méthyle dans 13 ml de benzène anhydre.

Le mélange a été chauffé jusqu'à dissolution puis on a ajouté 0,74 ml de pipéridine et 2,24 ml d'acide acétique glacial. La solution obtenue a été chauffée au reflux pendant 2 heures jusqu'à ce qu'il n'y ait plus de séparation d'eau. Après refroidissement, le mélange a été dilué par du benzène, lavé avec 20 ml d'acide chlorhydrique à 5%, du bicarbonate de sodium à 5% et de l'eau.

La phase organique a été décantée, séchée sur sulfate de magnésium anhydre et évaporée sous vide, donnant un produit huileux qui a été utilisé tel quel à l'étape suivante.

Rendement : 25 g (54%). Un échantillon a été purifié par recristallisation dans l'éthanolà 90°, donnant un solide blanc-jaune - PF = 73-74 °C.

b) 1,4-dihydro-2,6-diméthyl-4-(2',3'-méthylène dioxyphényl) - 3,5-pyridine dicarboxylate de méthyle.

On a dissous 10 g de $\alpha$-acétyl-$\beta$-(2,3-méthylènedioxyphényl) acrylate de méthyle dans 50 ml d'isopropanol chaud puis ajouté 4,3 g d'aminocrotonate de méthyle. Le mélange a été agité à la température ambiante pendant 48 heures. Le précipité solide a été filtré et le filtrat évaporé sous vide. On a obtenu un produit huileux qui a été recristallisé deux fois dans le méthanol chaud.

Rendement : 6 g (43%) - P.F. = 202 °C.

Exemple 4 : 1,4-dihydro-2,6-diméthyl-4-(2',3'-méthylènedioxyphényl)-3,5-pyridinedicarboxylate de méthyle et d'isobutyle

a) $\alpha$-acétyl -$\beta$-(2,3-méthylènedioxyphényl)-acrylate d'isobutyle.

Dans un ballon relié à un séparateur Dean-Stark contenant du benzène anhydre, on a introduit 10 g de 2,3-méthylènedioxybenzaldéhyde, 10,5 g d'acétoacétate d'isobutyle et 5 ml de benzène. Le mélange a été chauffé jusqu'à dissolution puis on a ajouté 0,26 ml de pipéridine et 0,80 ml d'acide acétique glacial.

La solution obtenue a été chauffée au reflux pendant 2 heures puis le mélange a été dilué par du benzène et lavé trois fois avec 20 ml d'acide chlorhydrique à 5%, du bicarbonate de sodium à 5% et de l'eau.

La phase organique a été séchée sur sulfate de magnésium anhydre puis le solvant éliminé sous vide, donnant un produit huileux qui a été cristallisé dans le n-hexane.

Le produit a été purifié par recristallisation dans l'isopropanol-n-hexane.

Rendement : 13,3 g (69%). P.F. = 62 - 64 ° C.

b) 1,4-dihydro-2,6-diméthyl-4-(2',3'-méthylènedioxyphényl)-3,5-pyridinedicarboxylate de méthyle et d'isobutyle.

On a dissous 10 g de α-acétyl -β-(2,3-méthylènedioxyphényl)-acrylate d'isobutyle dans 50 ml d'isopropanol chaud, puis ajouté 4 g d'aminocrotonate de méthyle. Le mélange a été agité 48 heures à la température ambiante puis le solvant a été éliminé sous vide.

Le produit huileux obtenu a été solidifié après ébullition dans le n-hexane et refroidissement.

Rendement : 10 g (80%). P.F. = 153 ° C.

Exemple 5 : 1,4-dihydro-2,6-diméthyl-4-(2',3'-méthylènedioxyphényl)-3,5-pyridinedicarboxylate d'isopropyle et de méthyle.

a) α-acétyl -β-(2,3-méthylènedioxyphényl)- acrylate de méthyle.

Cet intermédiaire est préparé selon la méthode décrite à l'exemple 3.

b) 1,4-dihydro-2,6-diméthyl-4-(2',3'-méthylènedioxyphényl) 3,5-pyridinedicarboxylate d'isopropyle et de méthyle.

On a dissous 5 g de α-acétyl-β-(2,3-méthylènedioxyphényl)-acrylate de méthyle dans 30 mld'isopropanol chaud, puis ajouté 2,8 g d'aminocrotonate d'isopropyle.

Le mélange a été agité pendant 48 heures à la température ambiante puis le solvant a été éliminé sous vide, donnant un produit huileux, solidifié par addition de n-hexane.

Le produit a été purifié par recristallisation dans le n-hexane-isopropanol.

Rendement : 3 g (40%).

Exemple 6 : 1,4-dihydro-2,6-diméthyl-4-(2',3'-méthylènedioxyphényl)-3,5-pyridinedicarboxylate d'isopropyle et d'éthyle.

a) α-acétyl -β-(2,3-méthylènedioxyphényl) acrylate d'éthyle.

Cet intermédiaire est préparé selon la méthode décrite dans l'exemple 1.

b) 1,4-dihydro-2,6-diméthyl-4-(2',3'-méthylènedioxyphényl)-3,5-pyridine dicarboxylate d'isopropyle et d'éthyle.

On a dissous 5 g de α-acétyl - β -(2,3-méthylènedioxyphényl) acrylate d'éthyle dans 50 ml d'isopropanol chaud puis ajouté 2,66 g d'aminocrotonate d'isopropyle. Le mélange a été chauffé pendant 48 heures à 40 ° C puis le solvant évaporé sous vide,donnant un solide qui a été recristallisé dans le n-hexane.

Rendement : 4,5 g (62%). P.F. = 165 ° C.

Les composés de l'invention ont fait l'objet de tests pharmacologiques mettant en évidence leurs propriétés inhibitrices des canaux calciques. Les résultats ont été comparés aux résultats obtenus avec des dérivés de 4-(polyméthoxy phényl) 1,4-dihydropyridine, considérés comme les composés de l'art connu les plus proches des composés de l'invention.

Les composés de référence suivants ont été préparés :

Référence 1 : 1,4-dihydro-2,6-diméthyl-4-(3' ,4' ,5'-triméthoxyphényl) 3,5-pyridinedicarboxylate de méthyle et d'éthyle.

a) α-acétyl-β-(3,4,5-triméthoxyphényl) acrylate d'éthyle.

Dans un ballon relié à un séparateur Dean-Stark contenant 100 ml de benzène, on a introduit une solution de 19,6g (0,11 mol.) de triméthoxybenzaldéhyde et 7 ml de benzène puis ajouté 13g (0,1 mol) d'acétoacétate d'éthyle.

On a agité le mélange à 60-70 ° C jusqu'à dissolution totale, puis ajouté 0,4 ml de pipéridine et 1,2 ml d'acide acétique.

On a chauffé au reflux 2 à 3 heures jusqu'à ce qu'il n'y ait plus de séparation d'eau. Après refroidissement, les cristaux ont été filtrés. On a obtenu 11 g.

Le filtrat a été dilué à l'éther puis lavé avec 50 ml d'acide chlorhydrique à 5%, 50 ml de bicarbonate de sodium à 5% et 50 ml d'eau. La phase organique a été séchée sur sulfate de magnésium anhydre, filtrée puis évaporée sous vide. On a obtenu 13 g supplémentaires.

Les deux fractions ont été recristallisées dans l'éthanol. Rendement : 75% - PF = 113,4 °C.

b) 1,4-dihydro-2,6-diméthyl-4-(3',4',5'-triméthoxyphényl) 3,5-pyridine dicarboxylate de méthyle et d'éthyle.

On a dissous 8g de α-acétyl -β-(3,4,5-triméthoxyphényl)-acrylate d'éthyle dans 50 ml d'isopropanol en chauffant doucement, puis ajouté 2,98g d'aminocrotonate de méthyle.

On a agité le mélange à la température ambiante pendant 48 heures puis filtré le solide obtenu. Le filtrat a été évaporé sous vide, donnant un semi-solide huileux qui a été recristallisé dans le méthanol.

Rendement : 5g (50%) - P.F. = 186 °C.

Référence 2: 1,4-dihydro-2,6-diméthyl-4-(3',4'-diméthoxyphényl)-3,5-pyridine dicarboxylate de méthyle et d'éthyle.

a) α-acétyl-β-(3,4-diméthoxyphényl)acrylate d'éthyle.

Dans un ballon relié à un séparateur Dean-Stark contenant du benzène anhydre, on a introduit 4,1 g (0,025 mol) de 3,4-diméthoxy benzaldéhyde et 3,25 g (0,025 mol) d'acétoacétate d'éthyle dans 5 ml de benzène anhydre.

On a chauffé le mélange jusqu'à dissolution totale puis ajouté 0,1 ml de pipéridine et 0,3 ml d'acide acétique glacial.

On a chauffé au reflux jusqu'à ce qu'il n'y ait plus de séparation d'eau.

Après refroidissement, le mélange a été dilué avec du benzène puis lavé deux fois avec 25 ml d'acide chlorhydrique à 5%, 25 ml de bicarbonate de sodium à 5% et de l'eau.

La phase organique a été séchée sur sulfate de magnésium anhydre et évaporée sous vide, donnant une huile utilisée sans autre purification à l'étape suivante.

Rendement : 5,8 g (85%).

b) 1,4-dihydro-2,6-diméthyl-4-(3',4'-diméthoxyphényl)-3,5-pyridinedicarboxylate de méthyle et d'éthyle.

On a dissous 4,8g de α-acétyl-β-(3,4-diméthoxyphényl) acrylate d'éthyle dans 25 ml d'isopropanol puis ajouté 2 g d'aminocrotonate de méthyle. On a agité le mélange pendant 48 heures à la température ambiante puis éliminé le solvant sóus vide.

On a obtenu un produit huileux qui a été recristallisé dans l'éthanol.

Rendement : 5 g (77%) - P.F. = 169 °C.

Référence 3 : 1,4-dihydro-2,6-diméthyl-4-(3',5'-diméthoxy-4'-hydroxyphényl) 3,5-pyridinedicarboxylate de méthyle et d'éthyle.

a) α-acétyl-β-(3,5-diméthoxy-4-hydroxyphényl)-acrylate d'éthyle.

Dans un ballon relié à un séparateur Dean-Stark contenant du benzène anhydre, on a introduit 9,1 g (0,05 mol) de 3,5-diméthoxy-4-hydroxy benzaldéhyde, 6,5 g.(0,05 mol) d'acétoacétate d'éthyle et 5 ml de benzène. On a chauffé jusqu'à dissolution totale puis ajouté 0,2 ml de pipéridine et 0,7 ml d'acide acétique glacial. La solution a été chauffée au reflux pendant 2 heures. Après refroidissement, le précipité formé a été filtré puis recristallisé dans l'éthanol.

Rendement : 11 g (75%).

b) 1,4-dihydro-2,6-diméthyl 4-(3',5'-diméthoxy-4'-hydroxyphényl)-3,5-pyridine dicarboxylate de méthyle et d'éthyle.

On a dissous 11 g de α-acétyl -β- (3,5-diméthoxy-4-hydroxyphényl)-acrylate d'éthyle dans 100 ml d'isopropanol puis ajouté 3,9 g d'aminocrotonate de méthyle. On a agité le mélange pendant 48 heures à la température ambiante.

Le solvant a été évaporé sous vide puis le composé huileux obtenu a été lavé trois fois avec 15 ml de n-hexane. Le solide obtenu a été recristallisé dans l'isopropanol.

Rendement : 8 g (55%). P.F. = 186 ° C.

Référence 4 : 1,4-dihydro-2,6-diméthyl-4-(2',3'-diméthoxyphényl)-3,5-pyridinedicarboxylate de méthyle et d'éthyle.

a) α-acétyl-β-(2,3-diméthoxyphényl) acrylate d'éthyle. La préparation de ce composé est décrite dans Organic Synthesis, Coll. vol. IV, page 408.

b) 1,4-dihydro-2,6-diméthyl-4-(2',3'-diméthoxyphényl)-3,5-pyridine dicarboxylate de méthyle et d'éthyle.

On a dissous 14 g de α-acétyl-β-(2,3-diméthoxyphényl) acrylate d'éthyle dans 50 ml d'isopropanol puis ajouté 5,8 g d'aminocrotonate de méthyle. Le mélange a été agité pendant 48 heures à la température ambiante, puis le solide obtenu a été filtré.

Par élimination du solvant sous vide, on a obtenu une seconde fraction.

Les deux fractions ont été recristallisées dans l'isopropanol.

Rendement : 11,5 g (61%). P.F. = 175 ° C.

Référence 5 : 1,4-dihydro-2,6-diméthyl-4-(3',5'-diméthoxyphényl)-3,5-pyridinedicarboxylate de méthyle et d'éthyle.

a) α-acétyl-β-(3,5-diméthoxyphényl) acrylate d'éthyle.

Dans un ballon relié à un séparateur Dean-Stark contenant du benzène, on a introduit 4,1 g de 3,5-diméthoxy benzaldéhyde et 3,25 g d'acétoacétate d'éthyle dans 5 ml de benzène anhydre.

On a chauffé le mélange doucement jusqu'à dissolution puis ajouté 0,1 ml de pipéridine et 0,3 ml d'acide acétique glacial.

La solution a été chauffée au reflux pendant 2 heures jusqu'à ce qu'il n'y ait plus de séparation d'eau.

Après refroidissement, le précipité obtenu a été filtré. Le filtrat a été lavé deux fois avec de l'acide chlorhydrique à 5% du bicarbonate de sodium à 5% et de l'eau puis le solvant a été éliminé sous vide, donnant un semi-solide huileux qui a été recristallisé dans l'éthanol.

Rendement total: 3,8 g (55%) - P.F. = 73 - 75 ° C.

b) 1,4-dihydro-2,6-diméthyl-4-(3',5'-diméthoxyphényl)-3,5-pyridinedicarboxylate de méthyle et d'éthyle.

On a dissous 3,5 g de α-acétyl -β- (3,5-diméthoxyphényl) acrylate d'éthyle dans 50 ml d'isopropanol chaud puis on a ajouté 2,25 g d'aminocrotonate de méthyle. On a chauffé le mélange pendant 48 heures à 40 ° C puis éliminé le solvant sous vide. Le composé huileux obtenu a été chauffé à l'ébullition dans du n-hexane. Par refroidissement, on a obtenu un précipité solide.

Rendement : 4 g (85%). P.F. = 122 ° C.

Les tests pharmacologiques ont été réalisés "in vitro" et "in vivo", selon des méthodes classiques.

1) Tests "in vitro"

Effets inhibiteurs des contractions induites, par le chlorure de potassium, dans l'aorte de rat.

Des rats Wistar de 220-250 g, ont été décapités puis leurs aortes ont été enlevées et placées dans un bain de 20 ml à 34 ° C, contenant une solution de Krebs de composition suivante (en mmol/l) : NaCl : 137; KCl : 2,7 ; $MgCl_2$, $6H_2O$ : 1,04; $CaCl_2$, $2H_2O$ : 0,8; $Na_2HPO_4$, $H_2O$ : 0,42; $NaHCO_3$ : 11,9 glucose : 5, oxygéné par $O_2$ (95%) + $CO_2$ (5%). (Furchgott and Bhadakrom, 1956).

Après une période de stabilisation de 45 minutes, sous 2 g de tension, les contractions maximales de l'artère ont été induites par addition de chlorure de potassium dans le bain, jusqu'à l'obtention d'une concentration finale de 80 mmol/l.

Après stabilisation des contractions, les composés de l'invention ou les composés de référence, ont été ajoutés, à des doses cumulatives, à des intervalles d'au moins 10 minutes pour permettre la stabilisation de la relaxation.

Des solutions de réserve à environ 1 mg/ml avaient été préparées par dissolution des composés de l'invention dans l'éthanol.

A partir de ces solutions, on avait obtenu des dilutions de $10^{-10}$ M à $10^{-6}$ M par addition de sérum physiologique.

Les concentrations inhibitrices 50 (C.I. 50) ont été déterminées par analyse de régression.

2) Tests "in vivo"

La pression sanguine systolique a été mesurée, sur la queue de rats spontanément hypertendus (RSH) et conscients, au moyen d'un brassard gonflable et d'un tensiomètre digital LE 5000 (Letica Instruments - Barcelone - Espagne).

Les mesures ont été réalisées avant l'administration du produit ainsi que 30 minutes, 1 heure, 2 heures, 6 heures et 24 heures après.

Pendant les mesures, les animaux étaient maintenus dans des cylindres de plastique pré-chauffés.

Les rats dont la pression sanguine était inférieure à 160 mm Hg ont été écartés de l'expérience. Chaque composé a été testé sur 5 animaux. Les composés de l'invention,ainsi que les composés de référence, ont été administrés par voie orale, sous forme de suspensions dans la gomme arabique à 5%.

Le tableau 1 montre l'activité in vitro des composés de l'invention sur les contractions induites dans l'aorte de rat par le chlorure de potassium (80 m M).

On peut voir que le composé 1, avec une $CI_{50}$ de $3.1 \times 10^{-9}$ M est beaucoup plus actif que les dérivés de diesters d'éthyle et de méthyle de référence. En particulier, il est au moins 30 fois plus actif que le composé de référence 4, composé de l'art connu, le plus proche.

Le composé 2 a montré une assez bonne activité.

Le composé 6, avec une $CI_{50}$ de $2,1 \times 10^{-9}$ M s'est révélé aussi actif que le composé 1.

Le tableau 2 montre l'activité in vivo sur la pression sanguine de rats RHS.

Dans ce test, le composé 1 (8mg/kg. P.O.) est 6 fois plus actif que le composé de référence 1.

Le composé 2, qui était assez actif in vitro, n'a révélé qu'un faible effet antihypertenseur alors que les composés 4 et 6 se sont montrés également actifs in vivo.

Tableau 1 : Activité in vitro des composés de l'invention sur l'aorte de rat dépolarisée au chlorure de potassium.

| Composé | | | Exemple | CI $\times 10^{-8}$ |
|---|---|---|---|---|
| R | $R_1$ | $R_2$ | | |
| 3',4',5'-triméthoxy | éthyl | méthyl | Ref. 1 | > 10 |
| 3',4'-diméthoxy | éthyl | méthyl | Ref. 2 | > 10 |
| 3',5'-diméthoxy 4'-hydroxy | éthyl | méthyl | Ref. 3 | > 10 |
| 2',3'-diméthoxy | éthyl | méthyl | Ref. 4 | > 10 |
| 3',5'-diméthoxy | éthyl | méthyl | Ref. 5 | 9,2 |
| | | | | |
| 2',3'-méthylènedioxy | éthyl | méthyl | 1 | 0,31 |
| 2',3'-éthylènedioxy | éthyl | méthyl | 2 | 4,5 |
| 2',3'-méthylènedioxy | méthyl | méthyl | 3 | 8,3 |
| 2',3'-méthylènedioxy | isobutyl | méthyl | 4 | 1,2 |
| 2',3'-méthylènedioxy | méthyl | isopropyl | 5 | 3,4 |
| 2',3'-méthylènedioxy | éthyl | isopropyl | 6 | 0,21 |

Tableau 2 : Action antihypertensive des composés de l'invention sur des rats spontanément hypertendus (RSH).

(dose = 8 mg/kg P.O - nombre d'animaux = 5)

| Composé | diminution maximum de la pression sanguine mm Hg | % |
|---|---|---|
| Ref. 1 | 9,8 | 5 |
| 1 | 65 | 33 |
| 2 | 12 | 7,2 |
| 4 | 48 | 23,5 |
| 5 | 10 | 5,2 |
| 6 | 62 | 32,4 |

## Revendications

**Revendications pour les Etats contractants suivants : BE, CH, DE, FR, GB, IT, LI, LU, NL, SE**

**1.** Dérivés de 1,4-dihydropyridine de formule générale (I):

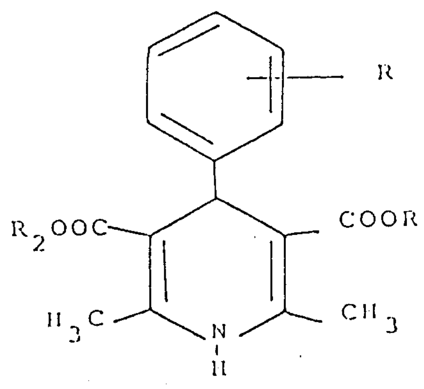

(I)

dans laquelle:

- R est un groupe 2,3-méthylènedioxy ou 2,3-éthylènedioxy, et
- $R_1$ et $R_2$ sont des groupes méthyle ou bien sont différents l'un de l'autre et choisis parmi les groupes méthyle, éthyle, isopropyle et isobutyle,

ainsi que leurs sels d'addition d'acides minéraux ou organiques pharmaceutiquement acceptables.

**2.** Dérivés selon la revendication 1, caractérisés en ce que $R^1$ est différent de $R^2$.

**3.** Selon les revendications 1 ou 2, le 1,4-dihydro-2,6-diméthyl-4-(2',3'-méthylènedioxyphényl)-3,5-pyridine dicarboxylate de méthyle et d'éthyle.

**4.** Selon les revendications 1 ou 2, le 1,4-dihydro-2,6-diméthyl-4-(2',3'-éthylènedioxyphényl)-3,5-pyridine dicarboxylate de méthyle et d'éthyle.

**5.** Selon les revendications 1 ou 2, le 1,4-dihydro-2,6-diméthyl-4-(2',3'-méthylènedioxyphényl)-3,5-pyridine

dicarboxylate de méthyle et d'isobutyle.

6. Selon les revendications 1 ou 2, le 1,4-dihydro-2,6-diméthyl-4-(2',3'-méthylènedioxyphényl)-3,5-pyridine dicarboxylate de méthyle et d'isopropyle.

7. Selon les revendications 1 ou 2, le 1,4-dihydro-2,6-diméthyl-4-(2',3'-méthylènedioxyphényl)-3,5-pyridine dicarboxylate d'éthyle et d'isopropyle.

8. Procédé de préparation des composés de formule générale (I), tels que revendiqués dans les revendications 1 ou 2, qui consiste à traiter un benzaldéhyde de formule (II):

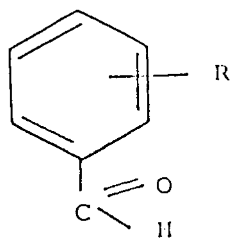

(II)

dans laquelle R est défini comme dans les revendications 1 à 2, par un ester de l'acide acétoacétique, de formule (III):

$H_3C\text{-}CO\text{-}CH_2\text{-}COOR_1$ (III)

dans laquelle $R_1$ est défini comme dans les revendications 1 à 2, puis à traiter l'ester de l'acide - $\alpha$-(acétyl)-$\beta$-(phényl substitué) acrylique obtenu, de formule (IV):

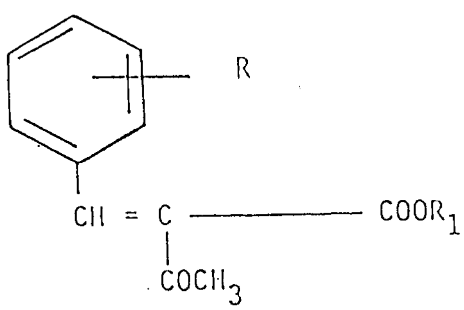

(IV)

par un ester de l'acide 3-aminocrotonique, de formule (V):

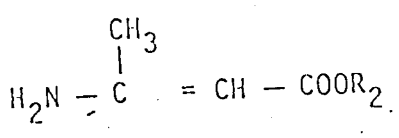

(V)

dans laquelle $R_2$ est défini comme dans les revendications 1 à 2, et éventuellement à traiter le produit obtenu de formule (I) par un acide minérale ou organique pharmaceutiquement acceptable.

9. Un composé selon l'une des revendications 1 - 7, utilisé comme médicament pour le traitement des troubles cardiovasculaires.

10. Composition pharmaceutique comprenant comme principe actif, un composé tel que revendiqué dans l'une des revendications 1 à 7, et un excipient pharmaceutiquement acceptable.

**Revendications pour l'Etat contractant suivant : AT**

**1.** Procédé de préparation des dérivés de 1,4-dihydropyridine de formule générale (I):

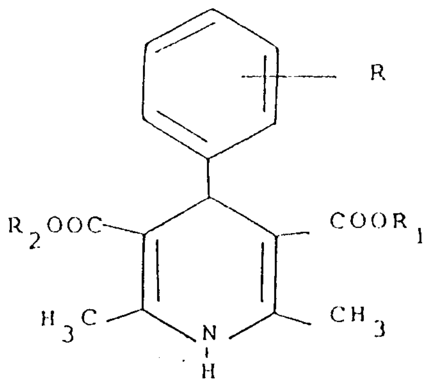

(I)

dans laquelle:

- R est un groupe 2,3-méthylènedioxy ou 2,3-éthylènedioxy, et
- $R_1$ et $R_2$ sont des groupes méthyle ou bien sont différents l'un de l'autre et choisis parmi les groupes méthyle, éthyle, isopropyle et isobutyle, méthoxyéthyle ou 2-pyridylméthyle,

ainsi que leurs sels d'addition d'acides minéraux ou organiques pharmaceutiquement acceptables, qui consiste à traiter un benzaldéhyde de formule (II)

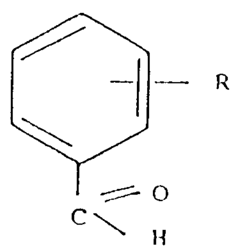

(II)

dans laquelle R est défini comme ci-dessus, par un ester de l'acide acétoacétique, de formule (III):

$H_3C - CO - CH_2 - COOR_1$ (III)

dans laquelle $R_1$ est défini comme ci-dessus, puis à traiter l'ester de l'acide $\alpha$-(acétyl)-$\beta$-(phényl substitué) acrylique obtenu, de formule (IV)

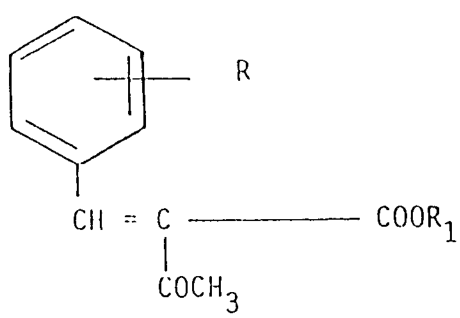

(IV)

par un ester de l'acide 3-aminocrotonique, de formule (V):

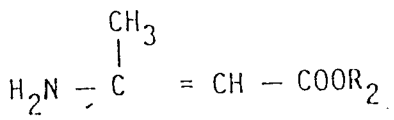

(V)

dans laquelle $R_2$ est défini comme précédemment, et éventuellement à traiter le produit obtenu de formule (I) par un acide minéral ou organique pharmaceutiquement acceptable.

**2.** Procédé de préparation selon la revendication 1, caractérisés en ce que $R^1$ est différent de $R^2$.

**3.** Selon les revendications 1 ou 2, la préparation de 1,4-dihydro-2,6-diméthyl-4-(2',3'-méthylènedioxyphényl)-3,5-pyridine dicarboxylate de méthyle et d'éthyle.

**4.** Selon les revendications 1 ou 2, la préparation de 1,4-dihydro-2,6-diméthyl-4-(2',3'-éthylènedioxyphényl)-3,5-pyridine dicarboxylate de méthyle et d'éthyle.

**5.** Selon les revendications 1 ou 2, la préparation de 1,4-dihydro-2,6-diméthyl-4-(2',3,-méthylènedioxyphényl)-3,5-pyridine dicarboxylate de méthyle et d'isobutyle.

**6.** Selon les revendications 1 ou 2, la préparation de 1,4-dihydro-2,6-diméthyl-4-(2',3'-méthylènedioxyphényl)-3,5-pyridine dicarboxylate de méthyle et d'isopropyle.

**7.** Selon les revendications 1 ou 2, la préparation de 1,4-dihydro-2,6-diméthyl-4-(2',3'-méthylènedioxyphényl)-3,5-pyridine dicarboxylate d'éthyle et d'isopropyle.

**8.** Préparation d'une composition pharmaceutique comprenant comme principe actif un composé obtenu dans l'une des revendications 1 à 7 qui consiste à mélanger le composé et un excipient pharmaceutiquement acceptable.

**Claims**
**Claims for the following Contracting States : BE, CH, DE, FR, GB, IT, LI, LU, NL, SE**

**1.** 1,4-dihydropyridine derivatives of formula (I) :

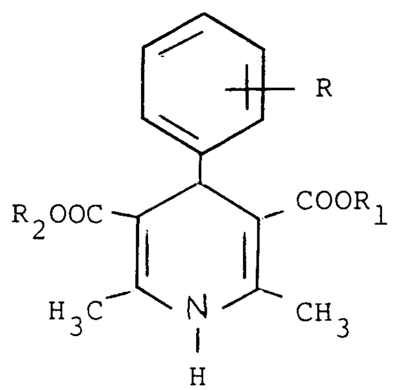

(I)

wherein :
- R is a 2,3-methylenedioxy or 2,3-ethylene-dioxy group, and
- $R_1$ and $R_2$ are both methyl groups or are different from each other and selected from methyl, ethyl, isopropyl and isobutyl groups,

and their pharmaceutically acceptable salts of addition with inorganic or organic acids.

**2.** Derivatives according to claim 1, characterized in that $R_1$ is different from $R_2$.

**3.** According to claims 1 or 2, 1,4-dihydro 2,6-dimethyl 4-(2',3'-methylenedioxyphenyl) 3-methoxy-carbonyl 5-ethoxycarbonyl pyridine.

**4.** According to claims 1 or 2, 1,4-dihydro 2,6-dimethyl 4-(2',3'-ethylenedioxy phenyl) 3-methoxy-carbonyl 5-ethoxycarbonyl pyridine.

**5.** According to claims 1 or 2, 1,4-dihydro 2,6-dimethyl 4-(2',3'-methylenedioxyphenyl) 3-methoxy-carbonyl 5-isobutoxycarbonyl pyridine.

**6.** According to claims 1 or 2, 1,4-dihydro 2,6-dimethyl 4-(2',3'-methylenedioxyphenyl) 3-methoxy-carbonyl 5-isopropoxycarbonyl pyridine.

**7.** According to claims 1 or 2, 1,4-dihydro 2,6-dimethyl 4-(2',3'-methylenedioxyphenyl) 3-ethoxy-carbonyl 5-isopropoxycarbonyl pyridine.

**8.** Process for the preparation of compounds of formula (I), according to claims 1 or 2, which consists in treating a benzaldehyde of formula (II) :

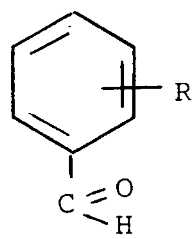

(II)

wherein R is defined as in claims 1 or 2,
with an acetoacetic acid ester, of formula (III) :

$H_3C-CO-CH_2-COOR_1$ (III)

wherein $R_1$ is defined as in claims 1 or 2,
then, in treating the resulting α- acetyl - β -(substitutedphenyl) acrylic acid ester, of formula (IV) :

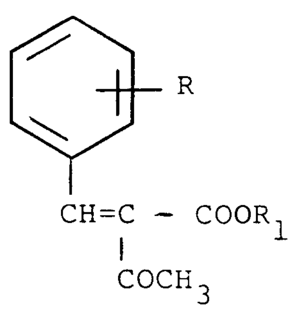

(IV)

with a 3-aminocrotonic acid ester, of formula (V):

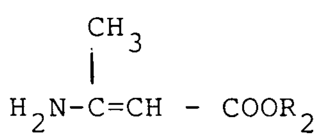

(V)

wherein $R_2$ is defined as in claims 1 or 2, and, if desired, in treating the resulting compound of formula (I) with a pharmaceutically acceptable inorganic or organic acid.

**9.** A compound according to anyone of claims 1 to 7, used as a medicament for the treatment of cardiovascular troubles.

**10.** A pharmaceutical composition comprising a compound as claimed in anyone of claims 1 to 7, as the active principle, and a pharmaceutically acceptable excipient.

**Claims for the following Contracting State : AT**

**1.** Process for the preparation of 1,4-dihydropyridine derivatives of formula (I) :

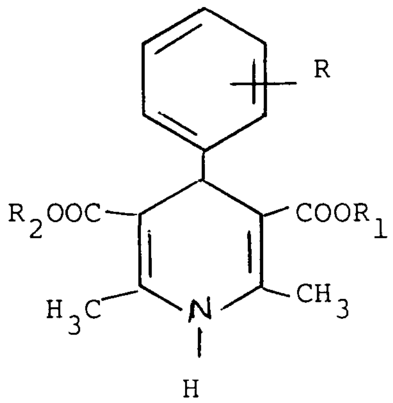

(I)

wherein :

- R is a 2,3-methylenedioxy or 2,3-ethylene-dioxy group, and
- $R_1$ and $R_2$ are both methyl groups or are different from each other and selected from methyl, ethyl, isopropyl and isobutyl groups,

and of their pharmaceutically acceptable salts of addition with inorganic or organic acids, which consists in treating a benzaldehyde, of formula (II):

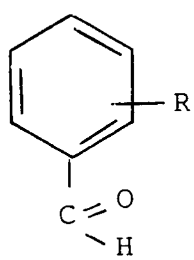

(II)

wherein R is defined as above,
with an acetoacetic acid ester,of formula (III) :

$H_3C\text{-}CO\text{-}CH_2\text{-}COOR_1$ (III)

wherein $R_1$ is defined as above,
then in treating the resulting $\alpha$-acetyl $\beta$ -(substituted phenyl) acrylic acid ester, of formula (IV):

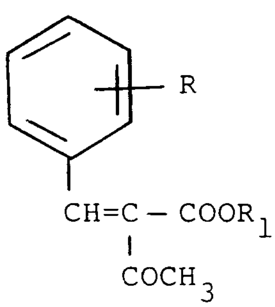

(IV)

with a 3-aminocrotonic acid ester, of formula (V):

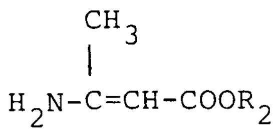

(V)

wherein $R_2$ is defined as above,

and, if desired, in treating the resulting compound of formula (I) with a pharmaceutically acceptable inorganic or organic acid.

**2.** Process according to claim 1, characterized in that $R_1$ is different from $R_2$.

**3.** According to claims 1 or 2, process for the preparation of 1,4-dihydro 2,6-dimethyl 4-(2',3'-methylenedioxyphenyl) 3-methoxycarbonyl 5-ethoxycarbonyl pyridine.

**4.** According to claims 1 or 2, process for the preparation of 1,4-dihydro 2,6-dimethyl 4-(2',3'-ethylenedioxyphenyl) 3-methoxycarbonyl 5-ethoxy-carbonyl pyridine.

**5.** According to claims 1 or 2, process for the preparation of1,4-dihydro 2,6-dimethyl 4-(2',3'-methylenedioxyphenyl) 3-methoxycarbonyl 5-isobutoxycarbonyl pyridine.

**6.** According to claims 1 or 2, process for the preparation of 1,4-dihydro 2,6-dimethyl 4-(2', 3'-methylenedioxy phenyl) 3-methoxycarbonyl 5-isopropoxycarbonyl pyridine.

**7.** According to claims 1 or 2, process for the preparation of 1,4-dihydro 2,6-dimethyl 4-(2', 3'-methylenedioxyphenyl) 3-ethoxycarbonyl 5-isopropoxycarbonyl pyridine.

**8.** Process for the preparation of a pharmaceutical composition comprising a compound obtained according to anyone of claims 1 to 7, as the active principle, which consists in mixing said compound with a pharmaceutically acceptable excipient.

**Patentansprüche**

**Patentansprüche für folgende Vertragsstaaten : BE, CH, DE, FR, GB, IT, LI, LU, NL, SE**

**1.** Derivate des 1,4-Dihydropyridins der allgemeinen Formel (I):

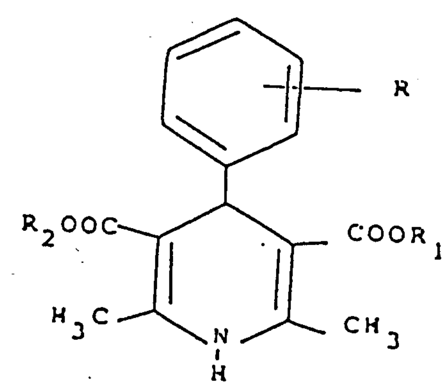

(I)

in der

- R eine 2,3-Methylendioxy- oder 2,3-Ethylendioxygruppe, und
- $R_1$ und $R_2$ Methylgruppen oder sich voneinander unterscheidende, aus den Gruppen Methyl, Ethyl, Isopropyl und Isobutyl ausgewählte Gruppen

bedeuten, sowie ihre pharmazeutisch verträglichen Additionssalze mit Mineralsäuren oder organischen Säuren.

**2.** Derivate gemäß Anspruch 1, dadurch gekennzeichnet, daß sich $R_1$ von $R_2$ unterscheidet.

**3.** 1,4-Dihydro-2,6-dimethyl-4-(2',3'-methylendioxyphenyl)-3,5-pyridindicarbonsäuremethyl- und -ethylester nach Anspruch 1 oder 2.

**4.** 1,4-Dihydro-2,6-dimethyl-4-(2',3'-ethylendioxyphenyl)-3,5-pyridindicarbonsäuremethyl- und -ethylester nach Anspruch 1 oder 2.

**5.** 1,4-Dihydro-2,6-dimethyl-4-(2',3'-methylendioxyphenyl)-3,5-pyridindicarbonsäuremethyl- und -isobutylester nach Anspruch 1 oder 2.

**6.** 1,4-Dihydro-2,6-dimethyl-4-(2',3'-methylendioxyphenyl)-3,5-pyridindicarbonsäuremethyl- und -isopropylester nach Anspruch 1 oder 2.

**7.** 1,4-Dihydro-2,6-dimethyl-4-(2',3'-methylendioxyphenyl)-3,5-pyridindicarbonsäureethyl- und -isopropylester nach Anspruch 1 oder 2.

**8.** Verfahren zur Herstellung von Verbindungen der allgemeinen Formel (L), wie in den Ansprüchen 1 oder 2 beansprucht, das darin besteht, daß man einen Benzaldehyd der Formel (II):

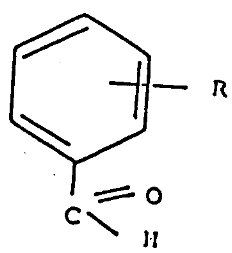

(II)

in der $R_1$ wie in den Ansprüchen 1 bis 2 definiert ist, mit einem Ester der Acetessigsäure der Formel (III)

$H_3C\text{-}CO\text{-}CH_2\text{-}COOR_1$ (III)

in der $R_1$ wie in den Ansprüchen 1 bis 2 definiert ist, behandelt, anschließend den erhaltenen Ester der $\alpha$-(Acetyl)-$\beta$-(substituiertes Phenyl)-acrylsäure der Formel (IV):

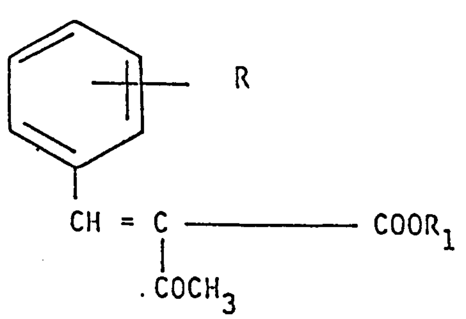

(IV)

mit einem Ester der 3-Aminocrotonsäure der Formel (V):

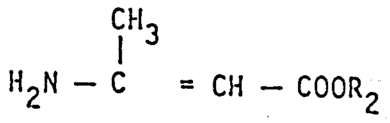

(V)

in der $R_2$ wie in den Ansprüchen 1 bis 2 definiert ist, behandelt und gegebenenfalls das erhaltene Produkt der Formel (L) mit einer pharmazeutisch verträglichen Mineral- oder organischen Säure behandelt.

**9.** Verbindung gemäß einem der Ansprüche 1 bis 7, verwendet als Medikament für die Behandlung von cardiovasculären Störungen.

**10.** Pharmazeutische Zusammensetzung, enthaltend als Wirkstoff eine Verbindung wie in einem der Ansprüche 1 bis 7 beansprucht sowie einen pharmazeutisch verträglichen Exzipienten.

**Patentansprüche für folgenden Vertragsstaat : AT**

**1.** Verfahren zur Herstellung von Derivaten des 1,4-Dihydropyridins der allgemeinen Formel (I):

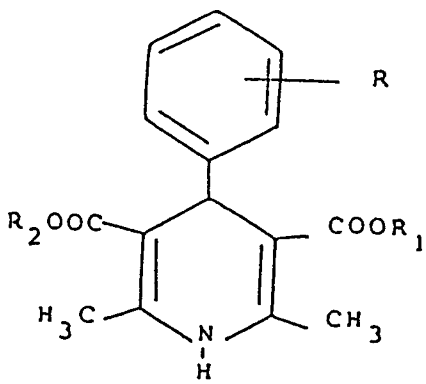

(I)

in der

- R eine 2,3-Methylendioxy- oder 2,3-Ethylendioxygruppe, und
- $R_1$ und $R_2$ Methylgruppen oder sich voneinander unterscheidende, aus den Gruppen Methyl, Ethyl, Isopropyl und Isobutyl ausgewählte Gruppen

bedeuten, sowie ihrer pharmazeutisch verträglichen Additionssalze mit Mineralsäuren oder organischen Säuren, das darin besteht, daß man einen Benzaldehyd der Formel (II)

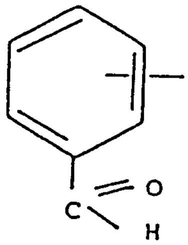

(II)

in der $R_1$ wie in den Ansprüchen 1 bis 2 definiert ist, mit einem Ester der Acetessigsäure der Formel (III)

$H_3C\text{-}CO\text{-}CH_2\text{-}COOR_1$ (III)

in der $R_1$ wie in den Ansprüchen 1 bis 2 definiert ist, behandelt, anschließend den erhaltenen Ester der

α-(Acetyl)-β-(substituiertes Phenyl)-acrylsäure der Formel (IV):

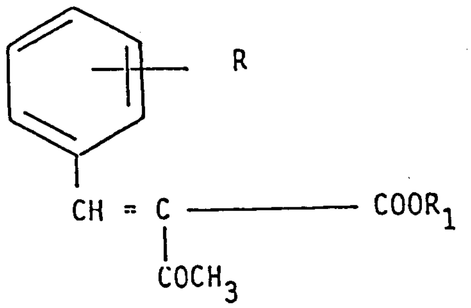

(IV)

mit einem Ester der 3-Aminocrotonsäure der Formel (V):

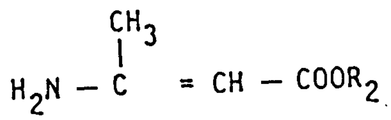

(V)

in der $R_2$ wie oben definiert ist, behandelt und gegebenenfalls das erhaltene Produkt der Formel (I) mit einer pharmazeutisch verträglichen Mineral- oder organischen Säure behandelt.

2. Verfahren zur Herstellung gemäß Anspruch 1, dadurch gekennzeichnet, daß $R_1$ von $R_2$ verschieden ist.

3. Herstellung von 1,4-Dihydro-2,6-dimethyl-4-(2',3'-methylendioxyphenyl)-3,5-pyridindicarbonsäuremethyl und -ethylester gemäß den Ansprüchen 1 oder 2.

4. Herstellung von 1,4-Dihydro-2,6-dimethyl-4-(2',3'-ethylendioxyphenyl)-3,5-pyridindicarbonsäuremethyl- und -ethylester gemäß den Ansprüchen 1 oder 2.

5. Herstellung von 1,4-Dihydro-2,6-dimethyl-4-(2',3'-methylendioxyphenyl)-3,5-pyridindicarbonsäuremethyl- und -isobutylester gemäß den Ansprüchen 1 oder 2.

6. Herstellung von 1,4-Dihydro-2,6-dimethyl-4-(2',3'-methylendioxyphenyl)-3,5-pyridindicarbonsäuremethyl- und -isopropylester gemäß den Ansprüchen 1 oder 2.

7. Herstellung von 1,4-Dihydro-2,6-dimethyl-4-(2',3'-methylendioxyphenyl)-3,5-pyridindicarbonsäureethyl und -isopropylester gemäß den Ansprüchen 1 oder 2.

8. Herstellung einer pharmazeutischen Zusammensetzung, enthaltend als Wirkstoff eine nach einem der Ansprüche 1 bis 7 erhaltene Verbindung, die darin besteht, daß man die Verbindung und einen pharmazeutisch verträglichen Exzipienten mischt.